# EUROPEAN PATENT SPECIFICATION

(11) **EP 4 500 187 B1**
(45) Date of publication and mention of the grant of the patent: **17.06.2026**
(21) Application number: 23714574.3
(22) Date of filing: 28.03.2023
(51) Int. Cl.: G01N 33/68, G01N 33/50, G01N 33/92

(54) **PREVENTING OVERTRAINING, OR DETERMINING THE APPROPRIATE AMOUNT OF EXERCISE**
VERHINDERUNG VON ÜBERTRAINING ODER ZUR BESTIMMUNG DES ANGEMESSENEN ÜBUNGSUMFANGS
PRÉVENTION D'UN SUR-ENTRAÎNEMENT, OU DE DÉTERMINATION DE LA QUANTITÉ D'EXERCICE APPROPRIÉE

(30) Priority: 29.03.2022 SE 2230095
(43) Date of publication of application: 05.02.2025
(73) Proprietor: Svexa Inc., Newark, DE 19713 (US)
(72) Inventor: Larsen, Filip, 153 91 Järna (SE)
(74) Representative: Novitas Patent AB
(86) International application number: PCT/EP2023/058065
(87) International publication number: WO 2023/186943

(56) References cited:
- WO-A1-2016/174454
- MICHAEL GLEESON: "Biochemical and immunological markers of over-training", JOURNAL OF SPORTS SCIENCE & MEDICINE, 1 June 2002 (2002-06-01), Turkey, pages 31 - 41, XP055202228, Retrieved from the Internet <URL:http://www.ncbi.nlm.nih.gov/pubmed/24688268>
- FINSTERER JOSEF ET AL: "Wet, volatile, and dry biomarkers of exercise-induced muscle fatigue", BMC MUSCULOSKELETAL DISORDERS, vol. 17, no. 1, 1 December 2016 (2016-12-01), XP093041812, Retrieved from the Internet <URL:https://bmcmusculoskeletdisord.biomedcentral.com/counter/pdf/10.1186/s12891-016-0869-2.pdf> DOI: 10.1186/s12891-016-0869-2
- LEE ELAINE C ET AL: "BRIEF REVIEW BIOMARKERS IN SPORTS AND EXERCISE: TRACKING HEALTH, PERFORMANCE, AND RECOVERY IN ATHLETES", J STRENGTH COND RES. 2017 OCT; 31(10): 2920-2937., 1 January 2017 (2017-01-01), XP093041825, Retrieved from the Internet <URL:https://www.ncbi.nlm.nih.gov/pmc/articles/PMC5640004/pdf/jscr-31-2920.pdf>
- SOL JOSEPH A. ET AL: "Application of a Novel Collection of Exhaled Breath Condensate to Exercise Settings", INTERNATIONAL JOURNAL OF ENVIRONMENTAL RESEARCH AND PUBLIC HEALTH, vol. 19, no. 7, 1 January 2022 (2022-01-01), pages 3948, XP093041814, Retrieved from the Internet <URL:https://www.ncbi.nlm.nih.gov/pmc/articles/PMC8997655/pdf/ijerph-19-03948.pdf> DOI: 10.3390/ijerph19073948
- IACOBAZZI VITO ET AL: "Citrate - new functions for an old metabolite", BIOLOGICAL CHEMISTRY, vol. 395, no. 4, 17 January 2014 (2014-01-17), BERLIN, DE, pages 387 - 399, XP093041844, ISSN: 1431-6730, DOI: 10.1515/hsz-2013-0271
- DATABASE Pubmed [online] 1 January 1989 (1989-01-01), KOWALCHUK JM ET AL: "The effect of citrate loading on exercise performance, acid-base balance and metabolism - PubMed", XP093041860, retrieved from https://pubmed.ncbi.nlm.nih.gov/2767067/ Database accession no. 2767067
- J CLIN ET AL: "Original Article Effects of Citric Acid and L-Carnitine on Physical Fatigue", NUTR, 1 November 2007 (2007-11-01), pages 224 - 230, XP055623495, Retrieved from the Internet <URL:https://www.ncbi.nlm.nih.gov/pmc/articles/PMC2243251/pdf/jcbn2007063.pdf>
- SATO SHOGO ET AL: "Atlas of exercise metabolism reveals time-dependent signatures of metabolic homeostasis", CELL METABOLISM, CELL PRESS, UNITED STATES, vol. 34, no. 2, 13 January 2022 (2022-01-13), pages 329, XP086947010, ISSN: 1550-4131, [retrieved on 20220113], DOI: 10.1016/J.CMET.2021.12.016
- MOREIRA LETÍCIA PARADA ET AL: "Detecting urine metabolites related to training performance in swimming athletes by means of Raman spectroscopy and principal component analysis", JOURNAL OF PHOTOCHEMISTRY AND PHOTOBIOLOGY B: BIOLOGY, ELSEVIER SCIENCE S.A., BASEL, CH, vol. 185, 22 June 2018 (2018-06-22), pages 223 - 234, XP085424564, ISSN: 1011-1344, DOI: 10.1016/J.JPHOTOBIOL.2018.06.013
- LONNEKE M JANSSENDUIJGHUIJSEN ET AL: "Adaptation of exercise-induced stress in well-trained healthy young men", EXPERIMENTAL PHYSIOLOGY, CAMBRIDGE UNIVERSITY PRESS, CAMBRIDGE, GB, vol. 102, no. 1, 11 December 2016 (2016-12-11), pages 86 - 99, XP071896939, ISSN: 0958-0670, DOI: 10.1113/EP086025
- PETIBOIS C ET AL: "Alterations of lipid profile in endurance over-trained subjects", ARCHIVES OF MEDICAL RESEARCH, INSTITUTO MEXICANO DEL SEGURO SOCIAL, MEXICO, MX, vol. 35, no. 6, 1 November 2004 (2004-11-01), pages 532 - 539, XP022760559, ISSN: 0188-4409, [retrieved on 20041231], DOI: 10.1016/J.ARCMED.2004.11.013
- REHMAN TAHNIAT ET AL: "Cysteine and homocysteine as biomarker of various diseases", FOOD SCIENCE & NUTRITION, vol. 8, no. 9, 12 August 2020 (2020-08-12), pages 4696 - 4707, XP093041820, ISSN: 2048-7177, Retrieved from the Internet <URL:https://onlinelibrary.wiley.com/doi/full-xml/10.1002/fsn3.1818> DOI: 10.1002/fsn3.1818

## Description

### Field of invention

The invention relates to exercise regulation methods, and in particular to a non-therapeutic method of preventing overtraining in a subject, or of determining the appropriate amount of exercise for a subject. A data collection method and a computer system configured to perform a health or performance optimization method is also disclosed.

### Background

Overwhelming evidence exists that regular exercise is associated with a longer health span and confers protection against a plethora of diseases. Recent guidelines therefore suggest adults should do at least 150 to 300 minutes a week of moderate-intensity, or 75 to 150 minutes a week of vigorous-intensity physical activity (1).

There is a debate regarding the dose-response relationship between the volume of exercise training and health outcomes. Larger volumes of exercise are generally associated with better protection against diseases but at very high exercise volume some of this protection seems to be lost (2).

The challenge with finding the appropriate exercise training dose is even more pronounced in athletes. A common strategy employed by athletes to stimulate adaptations to endurance exercise is to perform an overreaching (OR) phase, where repeated training stress induces fatigue and disrupts homeostasis and favorable adaptations is believed to follow upon recovery. If the OR phase does not result in improved performance and/or bring physiological negative outcomes, the OR phase is considered as non-functional (NFOR). Under certain conditions NFOR can develop into the more severe overtraining syndrome (OTS) that requires several months or years of rest to recover from (3).

A lot of effort has been put into research trying to find suitable markers for the optimal prescription of exercise training. The most widely used today is testosterone and cortisol concentrations, creatine kinase and markers of oxidative stress (TBARS, Malondialdehyde) or inflammation (C-reactive protein, IL-6 etc.) (4). All of these markers have low sensitivity or are unspecific, i.e. they indeed show some change with exercise training but they cannot discriminate between optimal training and too little/too much training, yet they are widely used.

There is an urgent need to find the optimal dose of exercise on an individual level. As the dose of exercise associated with the largest health and performance benefits is highly individual, general guidelines on how to individualize exercise has limited usefulness.

Hence, there is a need for an advisory system preventing overtraining in a subject, or of determining the appropriate amount of exercise for a subject. Gleeson et al. (J Sports Sci Med, 2002 Jun; 1(2): 31-41) discloses biochemical and immunological markers of over-training. However, citrate is not disclosed as one of the markers.

### Short description of the invention

The present invention is a system as defined in independent claim 1. Further embodiments are disclosed in the dependent claims. By measuring the metabolite(s), advice can be given if increasing or decreasing the training load will result in additional health or performance benefits. A warning system for avoiding overtraining is hence also provided.

As used herein in accordance with the invention, when referring to exercise for a subject, a habitually exercising subject is referred to. Said habitually exercising subject is a subject performing exercise or manual labour from twice weekly, up to and including several times per day.

The invention as disclosed herein thus relates to the chronic effects of exercise or manual labour, but not to the acute effects thereof.

The invention shall now be described with reference to the accompanying figures, which shall however not be seen as limiting the scope of the invention in any way whatsoever.

### Abbreviations used

BL: baseline
LT: light training
MT: moderate training
NFOR: non-functional overreaching
OR: overreaching
OT: overtraining
OTS: overtraining syndrome
RE: recovery after overtraining

### Short description of the Figures

Figure 1 shows concentrations of four metabolites during increasing amounts of exercise training.
Figure 2 shows that both mitochondrial function (Fig 2a) and glucose tolerance (Fig 2b) improved when training increased from baseline to moderate training, but were markedly reduced after overtraining.
Figure 3 shows the change in mitochondrial function by a respective change in relation to baseline, of 0 - 4 metabolites chosen from the group of alpha-hydroxybutyrate, beta-hydroxybutyrate, citrate (citric acid), and L-cysteine. The percentage change in mitochondrial function is calculated as ratio between pre- and post-exercise training period values.

### Detailed description of the invention

Finding the dose of exercise training that produces the best outcomes in terms of health or physical performance is notoriously difficult and large individual differences exist. By using the metabolite profiling of the present invention, the exercise training dose may be fine-tuned, and advice may be given on a personal level, as to whether a subject has done too little, optimal or excessive amounts of training. As used herein, training is used as an abbreviation for "exercise training".

The invention relates to finding the dose of exercise training that produces the best outcomes for a habitually exercising subject. Said habitually exercising subject is a subject performing exercise or manual labour from twice weekly, up to and including several times per day. Hence, the invention relates to assessments of the chronic response to exercise (see below).

A habitually exercising subject (human or animal) is a subject involved in resistance or endurance exercise, such as walking, running, cycling, swimming, cross-country skiing, rowing, canoeing, skating, dancing, weight-lifting, martial arts, or performing manual labour from twice weekly, up to and including several times per day.

The effects of exercise or manual labour may be divided into acute response and chronic response.

The acute response to exercise involves the liberation of energy from various sources, for example breakdown of fats or carbohydrates to provide energy for muscle contraction. It also involves a release of hormones and catecholamines to aid in the breakdown of substrates and to tune the cardiovascular and nervous system for physical activity. In summary, the acute response to exercise is a stress response that breaks down the body, i.e. is catabolic. The acute response lasts from the first seconds of exercise up to the end of exercise or a few hours (i.e. less than 3 hours) thereafter.

The chronic response to exercise, on the other hand, is the response of the body over several days, weeks or months to repeated bouts of acute exercise. If the exercise sessions are appropriately intense and interspersed with enough recovery, sleep and nutrition between each session, the chronic response can be positive and the body grows stronger or fitter over time. If the exercise sessions are too intense or if the recovery period is too short, the chronic response can be negative and the body will break down over time, i.e. overtraining occurs. Alternatively, if the training sessions are too light or occur too infrequently, the chronic response can also be negative, since the stimuli for positive adaptations are lacking.

In accordance with the invention, the acute response to exercise is not being measured, nor analyzed.

As relates to the method steps disclosed herein, the skilled person realizes that these may be consecutive steps. However, any other order still resulting in the end result as claimed are also within the scope of the claims.

In accordance with a first aspect of the invention, there is provided a system as defined by independent claim 1.

Said first aspect of the invention may find application in setting up an exercise scheme for a subject whose health status or performance shall be improved. Said subject may be human, horse, dog, camel, dromedary, bird, or cattle. When the subject is a human, said human may be an athlete, a member of the general public, or a recovering subject.

As used herein, the term "providing a sample" shall be construed in such a way that sample taking is not part of the invention as claimed. The sample is provided from a habitually exercising subject. Negative effects of exercise may be e.g. a resting heart rate more than 1 standard deviation above normal baseline, decreased maximal heart rate, lowered exercise performance, worse mood, worse cognitive function, orthostatic intolerance, muscle soreness, reduced ability to produce blood lactate during intense exercise, higher blood lactate levels during submaximal exercise and at rest, decreased libido, sleep disturbances, weight gain or weight loss, increased ratings of perceived exertion for a given work load, increased mental stress, slower recovery than expected after training, depression or lack of motivation, glucose intolerance, more frequent hypoglycemic episodes, increased glucose variability, increased susceptibility to infections or injuries.

A subject not experiencing negative effects of exercise would not experience any pronounced individual status change(s) relating to the above individual factors.

A subject not experiencing any negative effects of exercise could also be a subject who has previously been engaged in too intense exercise and thus has experienced one or more of the typical effects listed above, but who has reduced the training load to recover, or is trying to recover.

Measuring metabolite(s) concentration(s) may be by use of mass spectrometry, HPLC/UPLC (high/ultra-high performance liquid chromatography), enzymatic assays using spectroscopic methods, ELISA (Enzyme-linked immunosorbent assays), radio-immunoassay, fluoro-immunoassay, electrochemical sensors including impedance spectroscopy, potentiometric sensors, cyclic voltammetry, ion-sensitive field-effect transistors (ISFETs), semiconductor field-effect transistors, methods utilizing ionic strength, pH-based methods, any electronic or chemical devices or biosensors (so-called wearables) that measure metabolites in the interstitial fluid or epidermis while attached on the skin, and methods including nanotechnology or microfluidics sensors. This list shall be seen as non-exhaustive, and merely exemplifies known analytical methods that may be made use of in accordance with the invention. The skilled person, having average skills in analytical methods suitable for analyzing metabolites, is well equipped to choose, and set up a suitable analytical method.

The metabolite citrate is an intermediate in the citric acid cycle, a central metabolic pathway for animals, plants, and bacteria. Citrate synthase catalyses the condensation of oxaloacetate with acetyl CoA to form citrate. Citrate then acts as the substrate for aconitase and is converted into aconitic acid. The cycle ends with regeneration of oxaloacetate. This series of chemical reactions is the source of two-thirds of the food-derived energy in higher organisms. It is known that aconitase is inhibited after periods of very intense exercise, and that citrate can accumulate in muscle tissue after intense exercise. Citrate is exported from the mitochondria to the cytoplasm by the transporter protein m-Indy. Dietary sources of citrate (mainly originating from citrus fruits and soft drinks) can be imported into the cell from the plasma membrane citrate transporter, but no export of mitochondrial derived citrate to blood or other body fluids has been shown. Increases in blood citrate levels can therefore constitute a mitochondrial stress signal during overtraining, i.e., when mitochondrial function is hampered.

The metabolite beta-hydroxybutyrate (BHB) is a ketone body that is synthesized in the liver under conditions of starvation or restricted carbohydrate availability. BHB levels increases from 0.1-0.3 uM under normal conditions to ~1mM after 3 days of fasting. The increase is normally highly correlated with an increase in plasma free fatty acid concentrations. It has been found that under conditions of overtraining, BHB increases despite normal free fatty acid levels and despite a high carbohydrate availability. This makes BHB a suitable marker for e.g., overtraining.

The metabolite alpha-hydroxybutyrate is produced in mammalian tissues that catabolize L-threonine or synthesize glutathione. Oxidative stress or detoxification demands can dramatically increase the rate of hepatic glutathione synthesis.

Under metabolic stress conditions, supplies of the metabolite L-cysteine for glutathione synthesis becomes limiting, so homocysteine is diverted from the transmethylation pathway forming methionine into the trans-sulfuration pathway forming cystathionine. Alpha-hydroxybutyrate is released as a by-product when cystathionine is cleaved to cysteine that is incorporated into glutathione. In line with this, it has been found that alpha-hydroxybutyrate increases and cysteine decreases after a period with overtraining. Therefore, both of these metabolites (or indeed the ratio between alpha hydroxybutyrate and cysteine) can be used as a marker for overtraining.

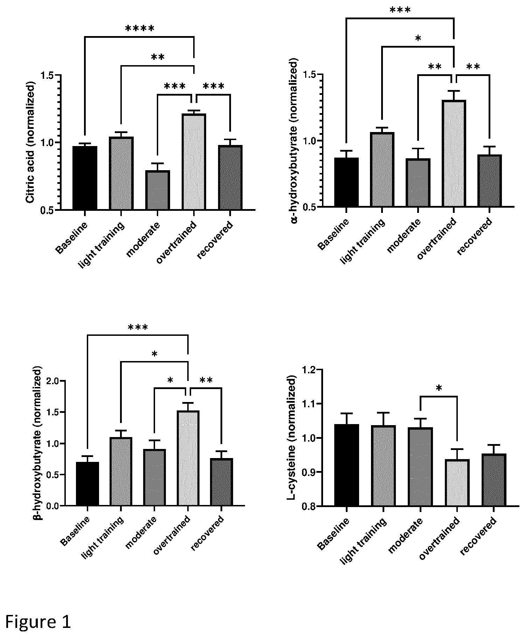

Figure 1 shows concentrations of the above four metabolites during increasing amounts of exercise training. After the overtraining period, concentrations of alpha-hydroxybutyrate (or alpha-hydroxybutyric acid), beta-hydroxybutyrate (or beta-hydroxybutyric acid), citrate (or citric acid) are significantly higher compared to the other situations. L-cysteine is significantly lower after overtraining compared to baseline, light training and moderate training, but not compared with recovered after overtraining. The absolute values of all metabolites are highly individual and therefore the concentrations are normalized to the individual mean values, as regards all 5 training conditions.

Citrate may in accordance with the invention be used either alone or in combination with any or all of the others to detect overtraining/NFOR. Using more metabolites than one, up to the total four, will increase the precision of the deviations, and hence the precision of the results obtained.

As used herein, exercise intensity, exercise frequency and exercise volume define the total exercise workload and constitute the entirety of what can be increased or reduced, in accordance with the invention. Said parameters are also what may be adjusted in accordance with the invention, in a decision-making step.

Exercise intensity is defined as the percentage of maximal oxygen consumption (VO2max), alternatively the percentage of maximal load, weight, speed or power, that is utilized at any moment during an exercise session.

Exercise frequency is defined as the number of exercise sessions per week.

Exercise volume is defined as the total time of exercise, per week.

Determination of deviation(s) of concentration(s) from baseline(s) may in accordance with the invention be made using computer-related means, such as software. Such software may be integrated in a watch or a mobile application. Alternatively, said determination may be made by hand, without use of any technical means.

The decision in the decision-making step may be a decision based directly on the deviation(s) determined in the previous step. No medical staff, such as a medical practitioner or nurse, would be needed in any decision-making step(s) in accordance with the invention.

Disclosed but not claimed is a non-therapeutic method of preventing overtraining in a subject, comprising the steps of:
a) Providing a sample from an exercising subject not experiencing negative effects of exercise;
b) Measuring ex vivo the concentration(s) of one or more metabolite(s) from the group comprising or consisting of citrate, alpha-hydroxybutyrate, beta-hydroxybutyrate, and L-cysteine, in the sample provided, to obtain a baseline or baselines;
c) Providing a sample from the same subject as in a), after said subject's exercise intensity, exercise frequency and/or exercise volume has been increased;
d) Measuring ex vivo the concentration of the same metabolite(s) as in b);
e) Determining the deviation(s) of the concentration(s) in d) from the baseline(s);
f) Making a decision, based on the deviation(s) in e), as to increasing or decreasing the exercise intensity, exercise frequency, and/or exercise volume, or to stay at the same level as expressed in c)

Disclosed but not claimed is a non-therapeutic method of determining the appropriate amount of exercise for a subject, comprising the steps of:
a) Providing a sample from an exercising subject not experiencing negative effects of exercise;
b) Measuring ex vivo of the concentration(s) of one or more metabolite(s) from the group comprising or consisting of citrate, alpha-hydroxybutyrate, beta-hydroxybutyrate, and L-cysteine, in the sample provided, to obtain a baseline or baselines;
c) Providing a sample from the same subject as in a), after said subject's exercise intensity, exercise frequency and/or exercise volume has been increased;
d) Measuring ex vivo the concentration(s) of the same metabolite(s) as in b);
e) Determining the deviation(s) of the concentration(s) in d) from the baseline;
f) Making a decision, based on the deviation(s) in e), as to increasing or decreasing the exercise intensity, exercise frequency, and/or exercise volume, or to stay at the same level as expressed in c)

Disclosed but not claimed is a non-therapeutic method of determining the health or performance status of a subject, comprising the steps of:
a) Providing a sample from an exercising subject not experiencing negative effects of exercise;
b) Measuring ex vivo the concentration(s) of one or more metabolite(s) from the group comprising or consisting of citrate, alpha-hydroxybutyrate, beta-hydroxybutyrate, and L-cysteine, in the sample provided, to obtain a baseline or baselines;
c) Providing a sample from the same subject as in a), after said subject's exercise intensity, exercise frequency and/or exercise volume has been increased;
d) Measuring ex vivo the concentration of the same metabolite(s) as in b);
e) Determining the deviation(s) of the concentration(s) in d) from the baseline.

Disclosed but not claimed is a non-therapeutic data collection method comprising the steps of:
a) Providing a sample from an exercising subject not experiencing negative effects of exercise;
b) Measuring ex vivo the concentration(s) of one or more metabolite(s) from the group comprising or consisting of citrate, alpha-hydroxybutyrate, beta-hydroxybutyrate, and L-cysteine, in the sample provided, to obtain a baseline or baselines;
c) Providing a sample from the same subject as in a), after said subject's exercise intensity, exercise frequency and/or exercise volume has been increased;
d) Measuring ex vivo the concentration(s) of the same metabolite(s) as in b);
e) Determining the deviation(s) of the concentration(s) in d) from the baseline;
f) Feeding deviation data from step e) into a database.

Said database would be accessible for any subsequent uses, including but not limited to statistical analyses, or population studies.

Disclosed but not claimed is a non-therapeutic computer system configured to perform a health or performance optimization method, the method comprising the steps of:
a) Providing a sample from an exercising subject not experiencing negative effects of exercise;
b) Measuring ex vivo the concentration of one or more metabolite(s) from the group comprising or consisting of citrate, alpha-hydroxybutyrate, beta-hydroxybutyrate, and L-cysteine, in the sample provided, to obtain a baseline or baselines;
c) Providing a sample from the same subject as in a), after said subject's exercise intensity, exercise frequency and/or exercise volume has been increased;
d) Measuring ex vivo the concentration(s) of the same metabolite(s) as in b);
e) Determining the deviation(s) of the concentration(s) in d) from the baseline;
f) Optimizing the exercise intensity/volume, exercise frequency, and/or exercise volume, based on the deviation(s) in e).

Said method may be automized in such a way that a reading is readily available from the computer system and/or software and/or mobile application.

The provision of a sample may be carried out by hardware not making use of any invasive means. Provision of a sample may be concomitant with analyzing the metabolite(s). Choosing and utilizing appropriate equipment to such end is well within the skills of the skilled person.

In step c) said subject's exercise intensity, exercise frequency and/or exercise volume has been increased during a time period of at least 5, 7, 10, or 14 consecutive days. A measurement is provided once a day, or once every two days. As an alternative, a sample may be provided for measurement on any of days 5, 7, 10, or 14, as counted from the first day of increased exercise. When there are several measurements available, these may be pooled for an average value to be calculated. Such an average value would increase the precision of the method.

The time period of increased exercise intensity, exercise frequency and/or exercise volume is compared with value(s) from a baseline time period of 5, 7 or 14 days immediately preceding said time period of increased exercise. A measurement is provided once a day, or once every two days. As an alternative, a sample may be provided for measurement on any of days 5, 7, 10, or 14, as counted backwards from the first day of increased exercise. When there are several measurements available, these may be pooled for an average value to be calculated. Such an average value would increase the precision of the method.

The time periods of 5, 7 or 14 days, both as regards the period of exercise increase, and the baseline period, aid in ensuring that only chronic responses to exercise are being measured and analyzed.

In accordance with the invention, each sample is in the form of blood, urine, sweat, interstitial fluid, saliva or exhaled breath condensate, preferably exhaled breath condensate. In steps a) and c), it is preferable to use the same kind of sample, when using the method, to facilitate the subsequent determination of deviation(s).

In accordance with the invention, it is advantageous to standardize the sampling of the body fluids. Ideally, urine, saliva, sweat, blood, interstitial fluid, or exhaled breath condensate should be collected after an overnight's fasting. A carbohydrate-rich meal shall preferably be consumed in the evening before body fluid collection. Ideally, a training session that the subject is accustomed to is performed in the afternoon on the day before body fluid collection.

In one embodiment, the samples provided in steps a) and c) are provided in the morning, e.g. after 5 am, after the subject has fasted from midnight, and within 72 h from the last time the exercising subject exercised. In a preferred embodiment, the samples provided in steps a) and c) are provided in the morning, e.g. after 5 am, after the subject has fasted from midnight, and at least 3 hours after the last time the exercising subject exercised, and within 72 h from the last time the exercising subject exercised. Providing the samples in the morning after an overnight fast and several hours after the last session of exercise is a way of ensuring that any acute response to exercise is not being measured and assessed.

In one embodiment, the samples in a) and c) are provided from a subject who has, on the day immediately preceding the day of providing said sample, eaten at least 3 grams of carbohydrates per kg bodyweight. Carbohydrate intake lower than 3 grams per kg bodyweight at that meal might give false positive readings on both BHB and citrate levels.

In accordance with the invention, as applicable, deviation(s) of any of the metabolite(s) concentration(s) from baseline(s) amounting to at least a 10% increase for citrate, at least a 15% increase for alpha-hydroxybutyrate, at least a 15% increase for beta-hydroxybutyrate, and below 95% of baseline for L-cysteine, indicates that the exercise intensity or exercise frequency should be reduced. The minimum changes in relation to baseline as herein described is defined as the "cut-off" value being exceeded.

In a preferred embodiment of the invention, as applicable, deviation(s) of any of the metabolite(s) concentration(s) from baseline(s) amounting to at least a 15% increase for citrate, at least a 22% increase for alpha-hydroxybutyrate, at least a 25% increase for beta-hydroxybutyrate, and below 95% of baseline for L-cysteine, indicates that the exercise intensity or exercise frequency should be reduced. The minimum changes in relation to baseline as herein described is defined as the "cut-off" value being exceeded.

In accordance with the invention, an advisory system is provided, wherein when none of the metabolites exhibit a change in relation to baseline as expressed herein, exercise intensity or exercise frequency or exercise volume should be increased, when 1-2 of the metabolites exhibit a change as expressed, exercise should be maintained at the same level, and when 3-4 of the metabolites exhibit a change as expressed, exercise intensity or exercise frequency or exercise volume should be decreased.

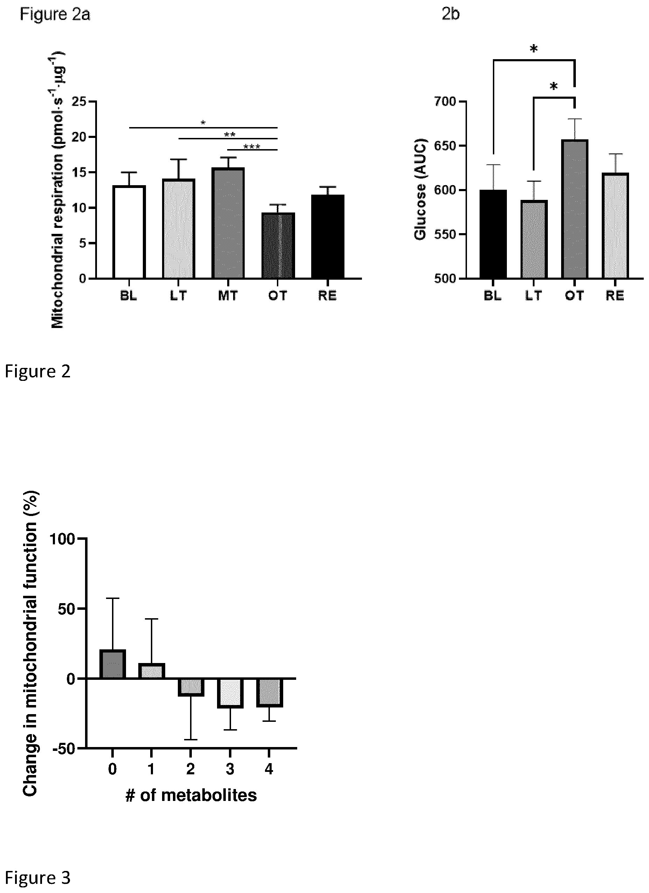

Figure 3 shows the change in mitochondrial function by a respective change in relation to baseline, of 0 - 4 metabolites chosen from the group of alpha-hydroxybutyrate, beta-hydroxybutyrate, citrate (citric acid), and L-cysteine. The percentage change in mitochondrial function is calculated as ratio between pre- and post-exercise training period values. From this graph it is clear that positive mitochondrial adaptations occur when no metabolite exceeds the cut-off value. When 1-2 metabolites exceed the cut-off values, neither positive nor negative adaptations occur. When 3 or 4 metabolites exceed the cut-off negative effects occur (n=42),

### Study behind the invention

11 human subjects were recruited to participate in the exercise training study. They were all healthy and conducted endurance and strength training on regular basis. Exclusion criteria were commitment to systematic endurance training of more than 5 hours a week as well as regular high intensity interval training (HIIT). The subjects trained progressively harder for three weeks, followed by a recovery week.

Studied conditions: before training is baseline (BL) level; week 1 was light training (LT); week 2 was medium training (MT); week 3 was overtraining (OT); and week 4 was a recovery week (RE). Blood samples and muscle biopsies were collected at baseline and after each training week. The 4 metabolites of the invention were analyzed in blood plasma to find discriminating patterns between the 5 conditions.

To assess the health and performance outcomes after each training load, a glucose tolerance test was performed after each training week, and an assessment of mitochondrial function carried out. Glucose tolerance tests, being used to diagnose type 2 diabetes and increases in mitochondrial function, is a hallmark of positive exercise adaptations. As can be seen in Figure 2, both mitochondrial function (Fig 2a) and glucose tolerance (Fig 2b) improved when training increased from BL to MT but were markedly reduced after a week of OT. Hence, performance improvements stalled after OT, which is a clear indication of maladaptation and a confirmation that the subjects were overtrained or experienced negative effects of the exercise training load (5).

For each metabolite a cut-off value was established that was based on the change from the normal baseline value. For citrate, a concentration 10% or higher than the baseline is an indication that training intensity or volume should be reduced. For beta-hydroxybutyrate a concentration 15% or higher than the normal baseline is an indication that training intensity or volume should be reduced. For L-cysteine a concentration below 95% of baseline is an indication that training intensity or volume should be reduced. For alpha-hydroxybutyrate a concentration 15% or higher than the normal baseline is an indication that training intensity or volume should be reduced.

Exercise is a stressful event for the body and increases in a few of the 4 metabolites described above is expected after challenging, but not excessive, exercise sessions. Hence, an advisory system was developed, which is based on the number of metabolites that exceed the cut-off levels described hereinabove:

| Number of metabolites exceeding the cut-off level | Advice to adjust exercise training |
|---|---|
| 0 | Increase exercise training |
| 1-2 | Maintain exercise at the same level |
| 3-4 | Decrease exercise training |

The above advisory system is illustrated in Figure 3 showing the change in mitochondrial function when different numbers of metabolites exceed the respective cut-off levels.

The skilled person realizes that it would be possible to make adaptations to the invention disclosed herein, which would be well within the scope of the inventive concept.

### References

1. https://www.who.int/publications/i/item/9789240015128
2. O'Keefe et al. Training for Longevity: The Reverse J-Curve for Exercise. Mo Med. 2020 Jul-Aug; 117(4): 355-361.
3. Kreher et al,. Overtraining Syndrome: A Practical Guide Sports Health. 2012 Mar; 4(2)
4. Lee et al,. Biomarkers in Sports and Exercise: Tracking Health, Performance, and Recovery in Athletes. J Strength Cond Res. 2017 Oct; 31(10): 2920-2937.
5. Flockhart et al. Excessive exercise training causes mitochondrial functional impairment and decreases glucose tolerance in healthy volunteers. Cell Metab 2021 May 4;33(5):957-970.e6

## Claims

1. Non-therapeutic computer advisory system configured to perform steps e) and f) in a performance optimization method, the method comprising the steps of:
a) Providing a sample from an exercising subject not experiencing negative effects of exercise;
b) Measuring ex vivo the concentration of one or more metabolite(s) from the group comprising or consisting of citrate, or citrate in combination with any of alpha-hydroxybutyrate, beta-hydroxybutyrate, or L-cysteine, in the sample provided, to obtain a baseline or baselines;
c) Providing a sample from the same subject as in a), after said subject's exercise intensity, exercise frequency and/or exercise volume has been increased during a time period of at least 5 consecutive days, as compared to the day immediately preceding said time period, or when experiencing negative chronic response to exercise;
d) Measuring ex vivo the concentration(s) of the same metabolite(s) as in b);
e) Determining the deviation(s) of the concentration(s) in d) from the baseline;
f) Optimizing the exercise intensity/volume, exercise frequency, and/or exercise volume, based on the deviation(s) in e), wherein, as applicable, deviation(s) of any of the metabolite(s) concentrations from baselines amounting to at least a 10% increase for citrate, at least a 15% increase for alpha-hydroxybutyrate, at least a 15% increase for beta-hydroxybutyrate, and below 95% of baseline for L-cysteine, indicates that the exercise intensity or exercise frequency should be reduced.

2. Non-therapeutic computer system according to claim 1, wherein the samples provided in steps a) and c) are provided in the morning, after the subject has fasted from midnight, and within 72 h from the last time the exercising subject exercised.

3. Non-therapeutic computer system according to any of claims 1 - 2, wherein the samples in a) and c) are provided from a subject who has, on the day immediately preceding the day of providing said sample, eaten at least 3 grams of carbohydrates per kg bodyweight.

4. Non-therapeutic computer system according to any of claims 1 - 3, wherein each sample is in the form of blood, urine, sweat, interstitial fluid, saliva or exhaled breath condensate, preferably exhaled breath condensate.

5. Non-therapeutic computer system according to any of claims 1 - 4, wherein the subject is a human, horse, dog, camel, dromedary, bird, or cattle.

6. Non-therapeutic computer system according to claim 5, wherein the human is an athlete, a member of the general public, or a recovering subject.

7. Non-therapeutic computer system according to any of the preceding claims, wherein the measurements of the metabolite(s) in b) and d) are performed by way of mass spectrometry, HPLC/UPLC, enzymatic assays using spectroscopic methods, ELISA, RIA, or fluoro-immunoassay.

## Patentansprüche

1. Nicht-therapeutisches Computerberatungssystem, das dazu konfiguriert ist, die Schritte e) und f) in einem Leistungsoptimierungsverfahren durchzuführen, wobei das Verfahren die Schritte umfasst:
a) Bereitstellen einer Probe von einem trainierenden Subjekt, das keine negativen Auswirkungen des Trainings wahrnimmt;
b) Ex-vivo-Messen der Konzentration von einem oder mehreren Metaboliten aus der Gruppe, die Citrat oder Citrat in Kombination mit einem der Metaboliten Alpha-Hydroxybutyrat, Beta-Hydroxybutyrat oder L-Cystein umfasst oder daraus besteht, in der bereitgestellten Probe, um einen Ausgangswert oder Ausgangswerte zu erhalten;
c) Bereitstellen einer Probe desselben Subjekts wie in a), nachdem die Trainingsintensität, die Trainingshäufigkeit und/oder das Trainingsvolumen dieses Subjekts während eines Zeitraums von mindestens 5 aufeinanderfolgenden Tagen im Vergleich zu dem diesem Zeitraum unmittelbar vorhergehenden Tag erhöht wurde oder wenn eine negative chronische Reaktion auf das Training wahrgenommen wird;
d) Ex-vivo-Messen der Konzentration(en) des/der gleichen Metaboliten wie in b);
e) Bestimmen der Abweichung(en) der Konzentration(en) in d) von dem Ausgangswert;
f) Optimieren der Trainingsintensität/des Trainingsvolumens, der Trainingshäufigkeit und/oder des Trainingsvolumens auf der Grundlage der Abweichung(en) in e), wobei gegebenenfalls eine Abweichung(en) einer der Metabolit(en)-Konzentrationen von den Ausgangswerten, die sich auf mindestens einen 10%igen Anstieg für Citrat, mindestens einen 15%igen Anstieg für Alpha-Hydroxybutyrat, mindestens einen 15%igen Anstieg für Beta-Hydroxybutyrat und unter 95% des Ausgangswertes für L-Cystein belaufen, anzeigt/anzeigen, dass die Trainingsintensität oder die Trainingshäufigkeit reduziert werden sollte.

2. Nicht-therapeutisches Computersystem nach Anspruch 1, wobei die in den Schritten a) und c) bereitgestellten Proben morgens bereitgestellt werden, nachdem das Subjekt seit Mitternacht gefastet hat, und innerhalb von 72 Stunden ab dem letzten Zeitpunkt, zu dem das trainierende Subjekt trainiert hat.

3. Nicht-therapeutisches Computersystem nach einem der Ansprüche 1 bis 2, wobei die Proben in a) und c) von einem Subjekt bereitgestellt werden, das an dem der Bereitstellung der Probe unmittelbar vorausgehenden Tag mindestens 3 g Kohlenhydrate pro kg Körpergewicht gegessen hat.

4. Nicht-therapeutisches Computersystem nach einem der Ansprüche 1 bis 3, wobei jede Probe in Form von Blut, Urin, Schweiß, interstitieller Flüssigkeit, Speichel oder Ausatemkondensat, bevorzugt Ausatemkondensat, vorliegt.

5. Nicht-therapeutisches Computersystem nach einem der Ansprüche 1 bis 4, wobei das Subjekt ein Mensch, ein Pferd, ein Hund, ein Kamel, ein Dromedar, ein Vogel oder ein Rind ist.

6. Nicht-therapeutisches Computersystem nach Anspruch 5, wobei es sich bei dem Menschen um einen Sportler, ein Mitglied der Allgemeinheit oder ein sich erholendes Subjekt handelt.

7. Nicht-therapeutisches Computersystem nach einem der vorhergehenden Ansprüche, wobei die Messungen des/der Metaboliten in b) und d) mittels Massenspektrometrie, HPLC/UPLC, enzymatischer Assays unter Verwendung spektroskopischer Methoden, ELISA, RIA oder Fluor-Immunoassay durchgeführt werden.

## Revendications

1. Système-conseil informatique non thérapeutique configuré pour réaliser les étapes e) et f) d'un procédé d'optimisation des performances, le procédé comprenant les étapes de :
a) fourniture d'un échantillon provenant d'un sujet faisant de l'exercice et ne ressentant pas les effets négatifs de l'exercice ;
b) mesure ex vivo de la concentration d'un ou plusieurs métabolites parmi le groupe comprenant ou consistant en citrate, ou en citrate en combinaison avec l'un quelconque parmi l'alpha-hydroxybutyrate, le bêta-hydroxybutyrate ou la L-cystéine, dans l'échantillon fourni, afin d'obtenir une ou plusieurs valeurs de référence ;
c) fourniture d'un échantillon provenant du même sujet que dans l'étape a), après que ladite intensité d'exercice, ladite fréquence d'exercice et/ou le volume d'exercice dudit sujet ont été augmentés pendant une période d'au moins 5 jours consécutifs, par rapport au jour précédant immédiatement ladite période, ou lorsqu'il présente une réponse chronique négative à l'exercice ;
d) mesure ex vivo de la ou des concentrations du ou des mêmes métabolites que dans l'étape b) ;
e) détermination du ou des écarts de la ou des concentrations de l'étape d) par rapport à la valeur de référence ;
f) optimisation de l'intensité/du volume d'exercice, de la fréquence d'exercice et/ou du volume d'exercice, en fonction du ou des écarts de l'étape e), le ou les écarts, le cas échéant, de l'une quelconque des concentrations de métabolites par rapport aux valeurs de référence représentant une augmentation d'au moins 10 % pour le citrate, une augmentation d'au moins 15 % pour l'alpha-hydroxybutyrate, une augmentation d'au moins 15 % pour le bêta-hydroxybutyrate, et moins de 95 % de la valeur de référence pour la L-cystéine, indiquant que l'intensité d'exercice ou la fréquence d'exercice doit être réduite.

2. Système-conseil informatique non thérapeutique selon la revendication 1, dans lequel les échantillons fournis aux étapes a) et c) sont fournis le matin, après que le sujet ait jeûné depuis minuit, et dans les 72 heures suivant la dernière fois que le sujet a fait de l'exercice.

3. Système-conseil informatique non thérapeutique selon l'une quelconque des revendications 1 et 2, dans lequel les échantillons dans les étapes a) et c) sont fournis par un sujet qui, la veille immédiate du jour de la fourniture dudit échantillon, a consommé au moins 3 grammes de glucides par kg de poids corporel.

4. Système-conseil informatique non thérapeutique selon l'une quelconque des revendications 1 à 3, dans lequel chaque échantillon est sous forme de sang, d'urine, de sueur, de liquide interstitiel, de salive ou de condensat d'air expiré, de préférence de condensat d'air expiré.

5. Système-conseil informatique non thérapeutique selon l'une quelconque des revendications 1 à 4, dans lequel le sujet est un humain, un cheval, un chien, un chameau, un dromadaire, un oiseau ou un bovin.

6. Système-conseil informatique non thérapeutique selon la revendication 5, dans lequel l'humain est un athlète, un membre du grand public ou un sujet en convalescence.

7. Système-conseil informatique non thérapeutique selon l'une quelconque des revendications précédentes, dans lequel les mesures du ou des métabolites dans les étapes b) et d) sont réalisées par spectrométrie de masse, CLHP/CLUP, dosages enzymatiques utilisant des méthodes spectroscopiques, ELISA, RIA ou dosage fluoro-immunologique.
